# DEMANDE DE BREVET EUROPEEN

(11) **EP 1 666 039 A1**
(43) Date de publication de la demande: **07.06.2006**
(21) Numéro de dépôt: 06003913.8
(22) Date de dépôt: 07.03.2003
(51) Int. Cl.: A61K 31/4439, A61K 9/107, A61P 37/00

(54) **Utilisation d'inhibiteurs de la L-alpha-lysophosphatidylcholine pour prévenir l'inflammation**

(30) Priorité: 11.03.2002 FR 0203499; 30.01.2003 FR 0301063
(62) Demande divisionnaire de: 03725275.6
(71) Demandeur: BIOMERIEUX, 69280 Marcy L'Etoile (FR); INSTITUT NATIONAL DE LA SANTE ET DE LA RECHERCHE MEDICALE, 75654 Paris Cédex 13 (FR)
(72) Inventeur: Lotteau, Vincent, 69390 Vourles (FR); André, Patrice, 69003 Lyon (FR)
(74) Mandataire: Maureau, Philippe

(57) **Abrégé**

L'invention concerne l'utilisation d'au moins un inhibiteur de L-α-lysophosphatidylcholine pour la fabrication d'un médicament pour la prévention d'une inflammation et/ou pour lutter contre une maladie inflammatoire et/ou une maladie autoimmune.

## Description

La présente invention concerne un procédé pour la différenciation de monocytes en cellules dendritriques matures selon lequel on dispose de monocytes dans un milieu approprié permettant leur différenciation et on additionne audit milieu le L-a-lysophosphatidylcholine. La présente invention concerne également l'utilisation d'au moins un inhibiteur de L-a-lysophosphatidylcholine pour la fabrication d'un médicament pour la prévention d'une inflammation et/ou pour lutter contre une maladie inflammatoire et/ou une maladie autoimmune.

Les cellules dendritiques sont impliquées dans le développement d'une réponse immune et dans l'initiation d'une réponse lymphocytaire T spécifique par reconnaissance, capture et présentation des antigènes notamment des agents infectieux (Steinman et al. 1997, Immuno. Rev., 156 : 25-37 ; Cella et al. 1997, Curr. Opin. Immunol., 9 : 10-16). Si les cellules dendritiques immatures captent et digèrent les antigènes des agents infectieux de façon très efficace, certains signaux, comme les agents bactériens ou les cytokines inflammatoires, peuvent les activer en induisant un processus de maturation, qui est l'étape initiale du déclenchement de la réponse immunitaire adaptative. En effet, au cours de cette activation, les cellules dendritiques acquièrent la capacité à migrer vers les organes lymphoïdes où se trouvent les lymphocytes T et la capacité à transmettre des signaux de co-stimulation indispensables à l'activation des lymphocytes T naïfs. Lors de cette maturation, les cellules dendritiques subissent des modifications fonctionnelles et phénotypiques telles que :
- une augmentation de molécules de surface impliquées dans l'activation des lymphocytes T (telles que CD40, CD80, CD83, CD86 et les molécules du complexe majeur d'histocompatibilité (CMH) de classe I et II),
- la production de cytokines proinflammatoires (telles que les interleukines IL-12, IL-1β, TNFα et IL-6)
- la diminution de leur capacité à capter et à apprêter l'antigène.

De part leurs capacités de développement d'une réponse immune et d'initiation d'une réponse lymphocytaire T spécifique, les cellules dendritiques matures présentent un intérêt thérapeutique particulier notamment dans les domaines de vaccination anti-infectieuse et anti-tumorale et de l'immunothérapie (Austin. 1998, Curr. Opin. Hematol. 5 : 3-15 ; Reise Sousa et al. 1999, Curr. Opin. Immunol. 11 : 392-399). Ces cellules dendritiques, de part leur capacité à induire une tolérance immunitaire, sont également une cible intéressante lorsque l'on souhaite inhiber la réponse immunitaire d'un patient, notamment pour lutter contre des maladies auto immunes ou inflammatoires.

*In vitro,* des cellules dendritiques matures peuvent être obtenues à partir de monocytes en culture. Ces monocytes sont *in vivo* des cellules circulantes qui, en traversant notamment la paroi vasculaire endothéliale, entrent au contact de facteurs environnementaux influençant leur devenir d'une façon encore méconnue. Schématiquement, on envisage alors trois possibilités pour ces monocytes :
- la sortie des tissus et le retour vers les ganglions lymphatiques,
- la différenciation en macrophages,
- la différenciation en cellules dendritiques immatures.
La première étape pour obtenir des cellules dendritiques matures à partir de monocytes en culture, consiste alors à induire la différenciation des monocytes en cellules dendritiques immatures par notamment l'interleukine IL-4 et le facteur GM-CSF (Granulocyte Macrophage-Stimulating factor). Au bout de 6 jours, 95% des cellules en culture sont des cellules dendritiques immatures.
La deuxième étape consiste ensuite en une induction de la maturation des cellules dendritiques immatures en cellules dendritiques matures par des agents exogènes, tels que des agents bactériens ou viraux. Ainsi, la protéine kpOmpA de *Klebsiella pneumoniae* est capable d'induire la maturation de ces cellules dendritiques immatures en cellules dendritiques matures (P. Jeannin et al., Nature Immunology, 2000, 1 : 502-509). On peut citer également la maturation des cellules dendritiques immatures en cellules dendritiques matures par d'autres molécules exogènes, telles que les lipopolysaccharides de la membrane bactérienne (Dichman et al, Journal of Cellular Physiology, 2000, 185 : 394-400). Toutefois, l'utilisation de molécules exogènes dérivées d'agents infectieux induit des problèmes sécuritaires et financiers, (effets secondaires directs ou indirects *in vivo ;* nécessité de purification très importante selon des exigences légales ou réglementaires très strictes...), rendant l'utilisation de ce type de molécules délicate dans le cadre de la vaccinologie et de l'immunothérapie.
Plus récemment, il a été décrit par Perrin-Cocon et al en 2001 (The Journal of Immunology, 167 : 3785-3791) que la production de cellules dendritiques matures à partir de monocytes en différenciation peut être induit par des molécules endogènes, telles que certaines lipoprotéines plasmatiques oxydées, et plus particulièrement les lipoprotéines de faible densité (LDL) oxydées. Toutefois, les LDL oxydés sont des particules complexes, composées de protéines, de triacylglycérols, de phospholopides, de cholestérol libre et estérifié, et sont, de ce fait difficiles à synthétiser artificiellement.

Il est également important de noter que si la stimulation de la différenciation des monocytes en cellules dendritiques est essentiels dans certaines applications thérapeutiques (lors d'une vaccination, d'une stimulation de la réponse immunitaire), il peut être également essentiel d'inhiber une telle différenciation des monocytes en cellules dendritiques, ou plus généralement d'inhiber la maturation des cellules dendritiques immatures, dans d'autres applications thérapeutiques, telles que lors d'une maladie auto immune ou inflammatoire. Il existe actuellement des traitements pour lutter contre l'inflammation, tels que la prise de corticoïdes, ou la prise d'aspirine. Le problème est que lorsque l'inflammation est chronique, de tels traitements ont des effets secondaires très néfastes pour le patient. Une prise prolongée de corticoïdes peut notamment engendrer un syndrome de Cuching au cours duquel on observe une déminéralisation, des fractures spontanées, du diabète. La prise prolongée d'aspirine peut engendrer quant à elle des ulcères à l'estomac. La présente invention se propose de résoudre les inconvénients de l'état de la technique en proposant également une molécule anti inflammatoire, facile à synthétiser et peu coûteuse qui inhibe la différenciation des monocytes en cellules dendritiques matures.

Ainsi, la présente invention se propose de résoudre les inconvénients de l'état de la technique en présentant une molécule proinflammatoire, molécule endogène, facile à synthétiser et peu coûteuse qui stimule la différenciation des monocytes en cellules dendritiques matures. La présente invention concerne également l'utilisation d'une émulsion lipidique comprenant des triglycérides, des phospholopides et du glycerol, telle que notamment l'Intralipid® pour inhiber la différenciation de monocytes en cellules dendritiques matures, de part une action directe, ou indirecte via une inhibition de l'action du L-α-lysophosphatidylcholine.

D'une façon surprenante, la présente invention concerne l'utilisation de L-α-lysophosphatidylcholine pour la différenciation de monocytes en cellules dendritiques matures *in vitro.*

Par L-α-lysophosphatidylcholine, on entend une molécule dont la formule est la suivante : 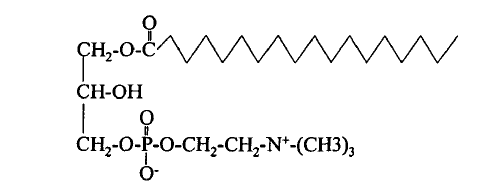 dans laquelle 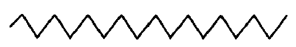 représente une chaîne longue d'acides gras saturés comprenant de 12 à 20 atomes de carbone, préférentiellement de 16 à 18.

En effet, une des molécules actives générées durant l'oxydation des LDL est le L-α-lysophosphatidylcholine, dénommé ci-après LPC. Toutefois, il a été montré que la fixation du LPC sur le récepteur G2A, qui est le récepteur de haute affinité du LPC, inhibe notamment la prolifération et l'activation des lymphocytes T, suggérant plutôt un rôle du LPC dans l'inhibition du déclenchement d'une réaction immunitaire (Carson & Lo, 2001, Science, 293 : 618-619). De plus, il a également été montré que de forte concentration de LPC (50µM) inhibe l'activité du facteur de transcription NF-κB (nuclear factor κB), connu pourtant pour être activé lors de la maturation des cellules dendritiques. De plus, le LPC est présent à forte concentration dans le plasma, suggérant qu'il ne serait pas actif dans le plasma, ce qui ne le prédispose pas à être choisi par l'homme du métier pour une utilisation thérapeutique.

L'invention concerne un procédé pour la différenciation *in vitro* de monocytes en cellules dendritriques matures selon lequel
A. on dispose de monocytes dans un milieu de culture approprié,
B. on induit la différenciation des monocytes en cellules dendritiques en présence d'un facteur de différenciation,
C. on additionne audit milieu de L-α-lysophosphatidylcholine et on obtient des cellules dendritiques matures.

Par milieu de culture approprié, on entend un milieu comprenant tous les éléments nécessaires à la viabilité des cellules. On peut citer à titre d'exemple le milieu RPMI 1640 et ses dérivés et tout milieu de culture bien connu de l'homme du métier. Ce milieu comprend notamment au moins un facteur de différenciation des monocytes en cellules dendritiques. Les facteurs de différenciation des monocytes en cellules dendritiques sont bien connus de l'homme du métier, et on peut citer notamment les cytokines telles que d'une manière non limitative l'interleukine IL-4, le facteur GM-CSF (Granulocyte Macrophage-Stimulating factor), l'IL-13, le TNF (tumor necrosis factor).

Lors de l'étape C) le L-α-lysophosphatidylcholine est additionné notamment audit milieu à une concentration finale dans le milieu comprise entre environ 10 et 80µM, préférentiellement entre environ 20 et 60 µM et avantageusement entre 30 et 50 µM. Toujours lors de l'étape C, le L-α-lysophosphatidylcholine est ajouté dans ledit milieu entre le 3^{ème} et 6^{ème} jour de différenciation des monocytes, préférentiellement entre le 4^{ème} et le 5^{ème} jour de différenciation des monocytes.

Selon un mode particulier de réalisation de l'invention, on ajoute en outre dans le milieu de culture, lors de l'étape C), au moins un agent biologique. Par agent biologique, on entend une molécule (ou un ensemble de molécules) qui est la cible d'une réponse immunitaire ou qui permet la synthèse de cette cible. Cet agent biologique peut ainsi être choisi parmi les antigènes de bactéries, de virus, de levures, de parasites, de champignons, les antigènes tumoraux, les lysats de cellules tumorales autologues et/ou héterologues. Par cellules tumorales autologues, on entend des cellules de tumeurs appartenant au sujet qui reçoit un médicament déterminé. Les cellules tumorales peuvent être obtenues par prélèvement de tissus cancéreux, notamment une biopsie ou résection chirurgicale. Par cellules tumorales hétérologues, on entend des cellules issues de tumeurs provenant d'un sujet différent de celui qui reçoit un médicament déterminé. L'utilisation de cellules hétérologues permet notamment d'obtenir un médicament permettant de traiter des patients atteints de cancer chez qui un prélèvement de cellules tumorales n'est pas possible. Cela permet aussi d'utiliser une source standard d'antigènes tumoraux. Par lysat cellulaire, on entend un mélange d'antigènes intra-cellulaires et/ou membranaires, obtenu par une lyse de cellules selon un protocole connu de l'homme du métier, telle qu'une lyse mécanique, chimique ou enzymatique. Cet agent biologique peut également être un acide nucléique qui code pour au moins un antigène choisi parmi les antigènes de bactéries, de virus, de levures, de parasites, de champignons, les antigènes tumoraux. Par antigène tumoral, on entend un antigène issu de cellules tumorales, tel qu'un peptide tumoral, notamment un peptide interagissant avec les molécules de classe I et présenté aux lymphocytes T CD8. On peut citer à titre non limitatif, les antigènes tumoraux suivants: MAGE-2, MAGE-3, MART, MUC-1, MUC-2, HER-2, GD2, carcinoembryonic antigen (CEA), TAG-72, ovarian-associated antigens OV-TL3 et MOV18, TUAN, alpha-feto protein (AFP), OFP, CA-125, CA-50, CA-19-9, renal tumor-associated antigen G250, EGP-40 (ou EpCAM), S100 (malignant meanoma-associated antigen), p53, prostate tumor-associated antigens (e.g., PSA et PSMA) et p21ras.
Ainsi, l'ajout de cet agent biologique dans le milieu de culture permet d'obtenir des cellules dendritiques matures qui permettent alors l'activation de lymphocytes T qui sont dirigées contre un antigène donné. Ces cellules dendritiques matures, obtenues *in vitro,* peuvent alors être *réinjectées in vivo.*
Dans un autre mode de réalisation de l'invention, on peut utiliser également, lors de l'étape C, un composé équivalent de L-α-lysophosphatidylcholine, qui est une molécule agissant selon les mêmes mécanismes d'action cellulaires que le L-α-lysophosphatidylcholine, c'est à dire par les mêmes récepteurs membranaires, notamment des récepteurs membranaires à protéines G, tels que les récepteurs G2A, GPR4, récepteur du PAF (facteur d'activation de plaquettes, platelet activating factor), et/ou par les mêmes récepteurs nucléaires tels que les récepteurs PPAR (peroxisome proliferator-activated receptor). Ce composé équivalent peut ainsi être notamment un agoniste des récepteurs précédemment cité (tel que notamment le O-methyl-PAF, le carbamyl-PAF, le 2-O-methyl-PAF), mais également le PAF lui même. Ce composé peut être également un agoniste des PPARδ tel que notamment le L165041 (Merck Research), GW-501516 (Glaxo), carboprostacyclin (Cayman) ou un antagoniste des PPARγ tel que notamment le bisphenol A diglycidyl ether (Sigma), le Diclofenac®. Ce composé équivalent de L-α-lysophosphatidylcholine peut être utilisé seul ou en synergie avec le L-α-lysophosphatidylcholine. Selon un mode préféré de réalisation de l'invention, le L-α-lysophosphatidylcholine est utilisé en synergie avec le facteur de d'activation de plaquettes (PAF).

L'invention concerne également un procédé pour l'activation de lymphocytes *T in vitro* selon lequel
A. on dispose de monocytes dans un milieu de culture approprié,
B. on induit la différenciation des monocytes en cellules dendritiques en présence d'un facteur de différenciation,
C. on additionne audit milieu de L-a-lysophosphatidylcholine et on obtient des cellules dendritiques matures,
D. on ajoute audit milieu un agent biologique, tel que défini précédemment, et on dirige les cellules dendritiques matures obtenues dans l'étape B contre ledit agent biologique,
E. on met en contact les cellules dendritiques matures dirigés contre l'agent biologique selon l'étape C avec des lymphocytes T et on obtient des lymphocytes T dirigés contre l'agent biologique.
L'étape C) peut être réalisée telle que décrit précédemment. Un tel procédé permet ainsi d'obtenir, *in vitro* des lymphocytes T dirigés contre un agent biologique donné, que l'on peut ensuite réinjecter, in vivo à un patient, notamment immunodéprimé.
L'invention concerne également un procédé pour la maturation de cellules dendritiques *in vitro* selon lequel
A. on dispose de cellules dendritiques immatures dans un milieu de culture approprié,
B. on additionne audit milieu de L-α-lysophosphatidylcholine et on obtient des cellules dendritiques matures.
L'homme du métier connaît bien les cellules dendritiques et leurs différents stades de maturation.
Lors de l'étape B) le L-α-lysophosphatidylcholine est additionné notamment audit milieu à une concentration finale dans le milieu comprise entre environ 10 et 80µM, préférentiellement entre environ 20 et 60 µM et avantageusement entre 30 et 50 µM.
Selon un mode particulier de réalisation de l'invention, on ajoute en outre dans le milieu de culture, lors de l'étape B), au moins un agent biologique tel que défini précédemment.
L'invention concerne également un milieu de culture caractérisé en ce qu'il comprend du L-α-lysophosphatidylcholine et au moins un facteur de différenciation tel que défini précédemment.
Dans un mode préféré de réalisation de l'invention, L-α-lysophosphatidylcholine est à une concentration finale dans le milieu comprise entre environ 10 et 80µM, préférentiellement entre environ 20 et 60 µM et avantageusement entre 30 et 50 µM.
Selon un mode particulier de réalisation de l'invention, le milieu de culture comprend en outre un agent biologique, tel que défini précédemment, qui est notamment un antigène de bactéries, de virus, de levures, de parasites, de champignons, de lysats de cellules tumorales autologues et/ou héterologues, un antigène tumoral, un acide nucléique qui code pour un antigène de bactéries, de virus, de levures, de parasites, de champignons, un acide nucléique qui code pour un antigène tumoral.
L'invention concerne également l'utilisation de L-α-lysophosphatidylcholine comme agent d'activation du système immunitaire. Par agent d'activation, on entend une molécule qui, dans une composition pharmaceutique, induit les effets d'une médication ou renforce ou complète les effets de la médication principale. Dans le cas d'une composition vaccinale, le L-α-lysophosphatidylcholine joue alors le rôle d'adjuvant qui stimule la réponse immunitaire de l'organisme hôte contre un antigène donné. Ainsi, l'invention concerne une composition vaccinale caractérisée en ce qu'elle comprend de L-α-lysophosphatidylcholine comme agent d'activation du système immunitaire, qui joue alors le rôle d'adjuvant et un agent biologique tel que défini précédemment, contre lequel on souhaite stimuler la réponse immunitaire du patient.
Selon un mode préféré de réalisation de l'invention, on utilise le L-α-lysophosphatidylcholine comme agent d'activation du système immunitaire pour la fabrication d'un médicament pour le traitement et/ou la prévention d'une infection d'origine bactérienne, virale, fongique, parasitaire ou provoquée par une levure, pour la fabrication d'un médicament pour le traitement et/ou la prévention des cancers.
Comme infection bactérienne, on peut citer notamment les infections induites par les staphylocoques, les mycobactéries, les bactéries du genre *Nisseria,* les légionelles, salmonelles...
Comme infection virale, on peut citer notamment les infections induites par le VIH (virus de l'immunodéficience humaine), les virus des hépatites, de la rougeole, rubéole, les poliovirus, flavivirus...
Comme infection fongique, on peut citer notamment l'aspergillose, candidose ...
Comme infection parasitaire, on peut citer notamment le paludisme, la leichmaniose...
Par cancer, on entend toutes les maladies dues à une multiplication anormale des cellules, et notamment de manière non limitatrice, les myélomes, lymphomes, leucémies, carcinomes du rein, du cerveau, du colon, de la prostate, du rectum, du pancréas, des ovaires, du poumon, du foie, du sein, les cancers cutanés choisi parmi les kératinomes et les carcinomes, les mélanomes.
Le médicament selon l'invention peut se présenter sous la forme d'une composition pharmaceutique en association avec au moins un excipient pharmaceutiquement acceptable bien connu de l'homme du métier. Dans les compositions pharmaceutiques selon l'invention, pour l'administration orale, sublingale, sous cutanée, intra musculaire, intra veineuse, topique, intratrachéale, rectale, transdermique, le L-α-lysophosphatidylcholine peut être administré sous forme unitaire d'administration ou en mélange avec des supports pharmaceutiques classiques, et destinés à une administration par voie orale, par exemple sous la forme d'un comprimé, d'une gélule, d'une solution buvable, etc, ou par voie rectale, sous la forme d'un suppositoire, par voie parentérale, en particulier sous la forme d'une solution injectable, notamment par voie intraveineuse, intradermique, sous cutanée, etc, selon des protocoles classiques bien connus de l'homme du métier. Pour l'application topique, on peut utiliser le L-α-lysophosphatidylcholine dans des crèmes, pommades, lotions, collyres.
Lorsqu'on prépare une composition solide sous forme de comprimés, on mélange le L-α-lysophosphatidylcholine avec un excipient pharmaceutiquement acceptable également nommé véhicule pharmaceutique, tel que la gélatine, l'amidon, le lactose, le stéarate de magnésium, le talc, la gomme arabique ou analogues. On peut enrober les comprimés de saccharose, d'un dérivé cellulosique, ou d'autres matières appropriées. On peut également les traiter de telles sortes qu'ils aient une activité prolongée ou retardée et qu'ils libèrent d'une façon continue une quantité prédeterminée de L-α-lysophosphatidylcholine. On peut également obtenir une préparation en gélules en mélangeant le L-α-lysophosphatidylcholine avec un diluant et en versant le mélange dans des gélules molles ou dures. On peut également obtenir une préparation sous forme de sirop ou pour l'administration sous forme de gouttes, dans laquelle le L-α-lysophosphatidylcholine est présent conjointement avec un édulcorant, un antiseptique, tel que notamment du méthylparaben et du propylparaben, ainsi qu'un agent donnant du goût ou un colorant approprié. Les poudres ou les granules dispersibles dans l'eau peuvent contenir le L-α-lysophosphatidylcholine en mélange avec des agents de dispersion ou des agents mouillants, ou des agents de mise en suspension, bien connus de l'homme du métier. Pour une administration parentérale, on utilise des suspensions aqueuses, des solutions salines isotoniques ou des solutions stériles et injectables qui contiennent des agents de dispersion, des mouillants pharmacologiquement compatibles, tels que notamment le propyléneglycol ou le butylèneglycol.
L'invention concerne également l'utilisation d'au moins un inhibiteur de L-α-lysophosphatidylcholine pour la fabrication d'un médicament pour la prévention d'une inflammation et/ou pour lutter contre une maladie inflammatoire et/ou une maladie autoimmune. Par inhibiteur de L-α-lysophosphatidylcholine, on entend une molécule (ou un ensemble de molécules) qui bloque l'activité inflammatoire et/ou immunostimulante du L-a-lysophosphatidylcholine, notamment en bloquant la différenciation des cellules dendritiques matures par le L-α-lysophosphatidylcholine. On entend également une molécule ayant des effets opposés à ceux du L-α-lysophosphatidylcholine, en modulant notamment le rapport PPARδ/PPARγ. On peut citer à titre indicatif une émulsion lipidique comprenant des triglycérides, des phospholipides et du glycerol, telle que notamment l'Intralipid®, le lipoïd E-80®, un agoniste des PPAR gamma. Les agonistes des PPAR gamma sont bien connus de l'homme du métier, et on peut citer d'une manière non limitatice, les molécules de la classe des thiazolidinediones, telles que notamment la ciglitazone, la troglitazone, la pioglitazone, la rosiglitazone.... De tels inhibiteurs pourraient ainsi *in vivo* être utilisés pour la fabrication d'un médicament pour diminuer une réponse inflammatoire, notamment articulaire, ou lors d'une greffe notamment. Selon un mode préféré de réalisation de l'invention, l'inhibiteur de L-α-lysophosphatidylcholine est une émulsion lipidique comprenant des triglycérides, des phopholopides et du glycerol. Préférentiellement, les triglycérides sont extraits d'huile végétale telle que notamment l'huile de soja. D'un manière encore plus préférentielle, l'émulsion lipidique comprend de 15 à 25 % d'huile de soja, préférentiellement 20% d'huile de soja, de 0,5 à 1,5 % de phospholipides d'oeuf, préférentiellement 1,2%, et de 1,8 à 2,6 % de glycérol, préférentiellement 2,2 % de glycérol.. Selon un autre mode préféré de réalisation de l'invention, l'inhibiteur est un agoniste des PPAR gamma tel que défini précédemment.
Comme maladie inflammatoire, on peut citer notamment la sarcoïdose, le lupus, la polyarthrite rhumatoïde, la spondylarthrite, l'uvéite...
Comme maladie auto immune, on peut citer notamment le diabète de type 1, la sclérose en plaques, le psoriasis, les hypersensibilités de contact, la polyarthrite rhumatoïde, la spondylarthrite ...

La figure 1 présente les courbes de titration donnant l'absorbance (Abs) en fonction du logarithme de la dilution du sérum de lot de souris recevant du LPC mélangé au lysozyme d'oeuf de poule (HEL, 1 mg/ml) ou du HEL seul.

Les exemples qui suivent sont donnés à titre explicatif et n'ont aucun caractère limitatif. Ils permettront de mieux comprendre l'invention.

### Exemple 1 - Différenciation de monocytes en culture en cellules dendritiques en présence ou en l'absence de L-α-lysophosphatidylcholine (LPC)

*Isolement, mise en culture et initiation de la différenciation des monocytes -* Les monocytes sont isolés de sang périphérique humain par une première centrifugation suivant le gradient de densité (620g ; 20 minutes) dans du Ficoll-Hypaque, suivie d'une deuxième centrifugation (770g ; 20 minutes) dans une solution à 50% en Percoll. Les monocytes sont ensuite purifiés par déplétion immunomagnétique (Dynal, Oslo, Norway), en utilisant un cocktail d'anticorps monoclonaux anti-CD 19 (hybridome 4G7) (Becton Dickinson, Francklin Lakes, NJ, USA), anti-CD3 (OKT3, American Type Culture Collection, Rockeville, MD) et anti-CD56 (NKH1, Beckman Coulter, Fullerton, CA, USA). Les monocytes ainsi obtenus sont alors purifiés à au moins 90%, comme montré par l'absence des marqueurs CD1a et la présence du marqueur CD14. La différenciation des monocytes en cellules dendritiques est initiée par 40ng/ml de GM-CSF (Granulocyte Macrophage-Colony Stimulating Factor) humain recombiné et 250U/ml d'interleukine IL-4 humain recombiné.

Les monocytes sont mis en culture dans le milieu RPMI 1640 (Life technologies, Rockeville, MD, USA) enrichi par 2mM de glutamine (Life technologies), 10mM d'Hepes (Life Technologies), 40ng/ml de gentamycine (Life technologies) et 10% de sérum de veau foetal dépourvu de lipoprotéines (LPDS, Sigma, St Quentin-Fallavier, France).

Notons que le milieu de culture présenté dans cet exemple est un milieu de culture LPDS. Des résultats comparables peuvent être obtenus par l'utilisation d'autres milieux de culture, tel qu'un milieu FCS, c'est à dire un milieu contenant 10% de sérum de veau foetal non dépourvu de lipoprotéines, ou pourraient être obtenus par l'utilisation de tout milieu de culture synthétique connus de l'homme du métier.

*Traitement des monocytes par le LPC -* 5 jours après le début de la différenciation des monocytes sont ajoutés dans le milieu de culture 40 µM de LPC pendant 24 heures (L-α-lysophosphatidylcholine ; Sigma ; St Quentin Fallavier, France). Des cellules contrôles sont également obtenues en l'absence de LPC dans le milieu de culture.

On obtient alors des cellules dites " contrôles " et des cellules dites " LPC ".

### Exemple 2 - Phénotype des cellules obtenues selon l'exemple 1

Les cellules utilisées dans cette analyse sont celles obtenues au 6ème jour de différenciation selon le protocole décrit dans l'exemple 1.

Le phénotype des cellules " contrôle " et " LPC " est analysé par cytométrie de flux sur un FACSCalibur (Becton Dickinson, Francklin Lakes, NJ, USA) par l'utilisation de conjugué FITC (isothiocyanate de fluorosceine) anti-CD14, anti-HLA-DR, anti-CD80 et conjugué PE (phycoerythrine) anti-CD1a, anti-CD83, anti-CD86, anti-CD40 (Beckman Coulter). Selon l'état de la technique, les monocytes ont préférentiellement un phénotype CD14+ CD1a-, les cellules dendritiques immatures ont préférentiellement un phénotype CD14- CD1a+ CD86-, les cellules dendritiques matures ont préférentiellement un phénotype CD 14- CD1a intermédiaire- CD86+.

Les résultats obtenus sont présentés dans le tableau 1.

**TABLEAU 1 - Expression des marqueurs CD83, HLA-DR et CD86 en absence (contrôle) ou en présence de LPC**

| | **CD83** | **HLA-DR** | **CD86** |
|---|---|---|---|
| **Contrôle** | 5,79 | 78,76 | 117,77 |
| **LPC** | 11,74 | 146,31 | 759,69 |

*Les cellules "LPC " obtenues après l'initiation de la différenciation des monocytes en présence de LPC, présentent un phénotype comparables à celui de cellules dendritiques matures, tel que le démontre notamment l'induction des marqueurs CD86, et l'augmentation de HLA-DR par comparaison au phénotype des cellules "contrôles* ".

### Exemple 3 - Capacité d'internalisation des cellules obtenues selon l'exemple 1

Les cellules " contrôle " et " LPC " utilisées dans cette analyse sont celles obtenues après 6 jours de différenciation selon le protocole décrit dans l'exemple 1. Ces cellules sont incubées à 37°C :
- pendant 30 minutes avec 1mg/ml de FITC-T70-Dextran (Sigma) afin d'estimer la capacité d'internalisation de ces cellules par endocytose,
- pendant 30 minutes avec 1mg/ml de Lucifer Yellow (ref. L0259, Sigma, St Quentin-Fallavier, France) afin d'estimer la capacité d'internalisation de ces cellules par pinocytose,
- pendant 3 heures avec carboxylate-modified yellow-green FluoSpheres (nom commercial, 0,45 µm, Molecular Probes, Leiden, The Netherlands) afin d'estimer la capacité d'internalisation de ces cellules par macropinocytose.
L'internalisation est stoppée sur glace par un tampon PBS froid contenant 0,1% de BSA (Bovine sérum Albumin, albumine sérique bovine) et 0,05% de NaN₃. Les cellules " contrôle " et " LPC " sont lavées 3 fois à 4°C dans ce même tampon et la fluorescence est quantifiée par FACScalibur (nom commercial, Becton Dickinson).
Comme le montre le tableau 2, les capacités d'internalisation par endocytose, pinocytose et macropinocytose sont fortement diminuées par l'ajout de LPC dans le milieu de culture au 5^{ème} jour de différenciation des monocytes par rapport aux cellules contrôles différenciées en l'absence de LPC.

**TABLEAU 2 - Capacité d'internalisation par endocytose, pinocytose et macropinocytose des monocytes différenciés en absence (contrôle) ou en présence de LPC**

| | **Endocytose** | **Pinocytose** | **Macropinocytose** |
|---|---|---|---|
| **Contrôle** | 100% | 100% | 100% |
| **LPC** | 53% | 49% | 66% |

*La réduction des capacités d'internalisation est une des caractéristiques des cellules dendritiques matures. Ces résultats montrent que en présence de LPC, les monocytes ont une forte tendance à se différencier en cellules dendritique matures.*

### Exemple 4 - Capacité des cellules obtenues selon l'exemple 1 à stimuler les lymphocites T

Les cellules " contrôle " et " LPC " utilisées dans cette analyse sont celles obtenues après 6 jours de différenciation selon le protocole décrit dans l'exemple 1.

Les lymphocytes T allogéniques naïfs sont isolés de sang périphérique humain. Les cellules mononuclées du sang périphérique sont isolées par centrifugation (600g, 20 minutes) selon le gradient de densité en présence de Ficoll-Hypaque. Après élimination des monocytes sur gradient de Percoll, les lymphocytes du sang périphérique se situent dans la fraction dense. Les lymphocytes T sont purifiés par déplétion immunomagnétique par l'utilisation d'un cocktail d'anticorps monoclonaux anti-CD 19 (anticorps 4G7) (Becton Dickinson, Francklin Lakes, NJ, USA), anti-CD16 (anticorps 3G8), anti-CD56 (anticorps NKH1), anti-glycophorin A (anticorps 11E4B7.6) et anti-CD14 (anticorps RMPO52) commercialisés par Beckman Coulter.
Les lymphocytes T purifiés sont cultivés dans des plaques de culture de 96 puits à fond plat avec les cellules " contrôle " ou " LPC ".

2x10⁵ cellules T sont cultivées dans 200µl de milieu de culture selon un rapport de 1:5, 1:10 ou 1:20 monocytes différenciés en présence ou non de LPC / cellules T (ratio DC/LT). Après 4 jours, 50µl du surnageant de culture sont utilisés pour déterminer la sécrétion d'IL-2 (interleukine 2) et d'IFNγ (interféron gamma) par un kit ELISA (Endogen, Woburn, MA, USA).

Comme le présente le tableau 3, la sécrétion d'IL2 et d'IFNγ par les lymphocytes T, exprimée classiquement selon le ratio cellules dendritiques/lymphocyte T, (ratio DC/LT) est fortement stimulée par les cellules provenant de la différenciation de monocytes en présence de LPC ajoutée 5 jours après l'initiation de la différenciation des monocytes (cellules "LPC "), par comparaison aux résultats contrôles (cellules " contrôles ").

**TABLEAU 3 - Stimulation de la sécrétion d'IL2 et d'IFNγ par lymphocytes T induite par les monocytes différenciés en absence (contrôle) ou en présence de LPC**

| **Ratio DC/LT** | | **0** | **0,05** | **0,1** | **0,2** |
|---|---|---|---|---|---|
| **Contrôle** | **IL-2** | 0 | 6 ± 8 | 15 ± 6 | 35 ± 11 |
| | **IFNγ** | 0 | 41±14 | 51±4 | 119±9 |
| **LPC** | **IL-2** | 0 | 23 ± 1 | 47 ± 26 | 173 ± 31 |
| | **IFNγ** | 0 | 73 ± 47 | 439 ± 108 | 795 ± 19 |

*Ces résultats montrent une augmentation des capacités des monocytes différenciés en présence de LPC à stimuler les lymphocytes T allogéniques, ce qui est une caractéristique des cellules dendritiques matures. A titre indicatif, des résultats comparables sont obtenus que le LPC soit dissous dans l'éthanol ou sous forme d'émulsion lipidique.*

### Exemple 5 - Propriétés des cellules obtenues selon l'exemple 1 : comparaison avec des cellules dendritiques matures obtenues par différenciation de monocytes en présence de LDL oxydés

L'objectif de cet exemple est de démontrer que les mécanismes d'action mis en jeu lors la différenciation des monocytes en cellules dendritiques matures en présence de LPC pourraient être différents de ceux mis en jeu lors de la différenciation des monocytes en cellules dendritiques matures en présence de LDL oxydés (Perrin-Cocon et al. The Journal of Immunology, 167 : 3785-3891, 2001).

Dans cet exemple sont utilisées des cellules " contrôle" et " LPC " telles qu'obtenues dans l'exemple 1, ainsi que des cellules "LDLox", obtenues tel que décrit dans la publication scientifique (Perrin-Cocon et al. The Journal of Immunology, 167: 3785-3891, 2001), c'est à dire après différenciation de monocytes en culture en présence de LDL oxydés ajoutés dans le milieu 5 jours après l'initiation de la différenciation.

Les inventeurs ont recherché si les inhibiteurs de l'action des LDL oxydés sur la différenciation des monocytes en cellules dendritiques matures inhibaient également l'action du LPC sur la différenciation des monocytes en cellules dendritiques matures.
Ainsi, une solution d'Intralipid® (50 µg/ml de phospholipides, Fresenius Kabi, Sèvres, France), ou une solution de molécules synthétiques de nature lipidique (lipoïd E-80®, Lipoïd Ag, Ludwigshafen, Allemagne, 50 µg/ml de phospholipides) ont été ajoutées dans le milieu de culture au 5^{ème} jour de différenciation. L'expression de CD86 des cellules "LPC" et " LDLox " en présence de ces inhibiteurs est analysée selon le protocole décrit dans l'exemple 2, et les résultats sont présentés dans le tableau 4.

**TABLEAU 4 - Inhibition (en %) de l'expression de CD86 des cellules LPC et LDLox par l'Intralipid® et le lipoïd E-80**

| | **Intralipid®** | **Lipoïd E-80** |
|---|---|---|
| **LDLox** | 88% ± 10 | 81% ± 14 |
| **LPC** | 86% ± 12 | 24% ± 19 |

L'ajout d'Intralipid® inhibe la différenciation des monocytes en cellules dendritiques matures par les LDL oxydés et par le LPC d'une façon comparable (inhibition de l'ordre de 80%).
Par contre, d'une façon surprenante, l'ajout de lipoïd E-80 induit une inhibition de l'expression des CD86 de 81% lorsque les monocytes sont cultivés en présence de LDL oxydés, alors que cette inhibition est de seulement 24% lorsque les monocytes sont cultivés en présence de LPC.
*Ces résultats suggèrent que les mécanismes d'action du LPC pour la différenciation des monocytes en cellules dendritiques matures seraient différents des mécanismes d'action des LDL oxydés.*

### Exemple 6 - Mécanismes cellulaires impliqués dans la différenciation des monocytes en cellules dendritiques matures en présence de LPC

Afin d'aller plus avant dans la recherche des mécanismes cellulaires de l'action du LPC dans la différenciation des monocytes en cellules dendritiques matures, les inventeurs ont recherché quels récepteurs pouvaient être impliqués.
Dans un premier temps, afin de déterminer si les récepteurs impliqués dans l'action du LPC lors de la différenciation des monocytes en cellules dendritiques matures appartenaient à la famille des récepteurs couplés à une protéine G, du PTX (toxine pertussis ; 100ng/ml), connu pour bloquer les protéines Gi est ajouté pendant 3 heures dans le milieu de culture. Le milieu de culture est ensuite changé et le LPC est ajouté tel que décrit dans l'exemple 1.
Les résultats présentés dans le tableau 5 montrent que la préincubation des monocytes par le PTX bloque l'augmentation de CD86 induite par le LPC, suggérant que l'action du LPC implique des récepteurs à protéines Gi.

**TABLEAU 5 - Inhibition de l'expression de CD86 des cellules " LPC " par le PTX**

| | **Induction de CD86** |
|---|---|
| **LPC** | 100% |
| **LPC + PTX** | 10% |

Ces récepteurs pourraient être notamment les récepteurs suivants :
- récepteur G2A (G2A-R) : il a été démontré récemment que le LPC était un ligand de forte affinité pour la protéine G2A, une protéine G couplé à un récepteur exprimé dans les lymphocytes. De plus, la stimulation de G2A par le LPC induit la phosphorylation d'une kinase extracellulaire (ERK1/2 : extracellular signal-related kinases). Cette phosphorylation peut d'ailleurs être observée lors de l'ajout de LPC dans le milieu de culture suggérant l'implication du récepteur G2A dans les différenciation des monocytes en cellules dendritiques matures par le LPC,
- récepteur PAF (PAF-R) comme décrit précédemment, certains effets du LPC pourraient impliquer le récepteur du PAF dans divers types de cellules,
- récepteur GPR4 : le LPC est également un ligand de la protéine GPR4, une autre protéine G couplé à un récepteur ayant une forte affinité pour le sphingosylphosphorylcholine.
Ensuite, les inventeurs ont recherché les facteurs de transcription impliqués dans le processus de maturation induit par la LPC. Afin de déterminer si les PPAR sont impliqués dans la maturation induite par la LPC, la capacité de liaison des deux facteurs de transcription PPARγ et PPARδ a été analysée par la technique de retard sur gel. Il existe 3 isotypes de récepteurs nucléaires PPAR (peroxisome proliferator-activated receptor) : α, γ et δ. Les PPARγ sont les cibles de molécules de la classe des thiazolidinediones comme la Ciglitizone, utilisées dans le traitement du diabète de type II. Les PPARδ ont une expression plus large et peuvent être répresseur des PPARγ. Les monocytes en cours de différenciation ont été incubés pendant deux heures avec une solution de LPC (40 µM) (cellules dites « LPC »). Des cellules dites « contrôle » sont également obtenues en l'absence de LPC dans le milieu de culture. Après récolte des cellules, les protéines nucléaires ont été extraites avec le kit Nuclear extract Kit (SIGMA). Les protéines nucléaires ont ensuite été mises au contact d'une sonde marquée radioactivement contenant un élément de réponse reconnu par les PPARs. Après migration sur un gel non dénaturant, une bande contenant PPARγ et un doublet contenant PPARδ ont été identifiés à l'aide d'anticorps par la technique de Supershift. L'activité de liaison des PPAR a été déterminée par mesure de l'intensité de ces bandes. Ces activités sont exprimées en pourcentage par rapport aux cellules dites « contrôle ».

**Tableau 6 - Modulation de l'activité des PPARγ et PPARδ par le LPC**

| | PPARγ | PPARδ |
|---|---|---|
| **Contrôle** | 100 % | 100 % |
| **LPC** | 0 % | 263 % |

Le traitement par LPC induit une forte réduction de l'activité de liaison de PPARγ pouvant conduire à une disparition totale et stimule fortement l'activité de PPARδ.

Les inventeurs ont ensuite utilisés un agoniste des PPARγ, la Ciglitizone. Une solution de Ciglitizone (Sigma, 50 µM) a été ajoutée dans le milieu de culture au cinquième jour de différenciation des monocytes, comme décrit dans l'exemple 1, 15 minutes avant l'ajout d'une solution de LPC (40 µM pendant 2 heures). Les cellules obtenues sont dites « LPC + Ciglitizone ». Des cellules dites « Ciglitizone » ont été obtenues selon le même protocole mais en l'absence de LPC et des cellules dites « LPC » ont été obtenues comme décrit dans l'exemple 1, en l'absence de ciglitizone.
L'activité des facteurs de transcription PPARγ et PPARδ a été mesurée dans ces cellules par la technique de gel retard, comme décrit ci-dessus.

**Tableau 7 - La Ciglitizone bloque l'inactivation de PPARγ et réduit l'augmentation de PPARδ induite par LPC**

| | PPARγ | PPARδ |
|---|---|---|
| **Contrôle** | 100 % | 100 % |
| **Ciglitizone** | 237 % | 106 % |
| **LPC + Ciglitizone** | 115 % | 147 % |

Ces résultats indiquent que la Ciglitizone qui active PPARγ, inhibe fortement l'effet du LPC sur les PPARγ et PPARδ, et bloque de ce fait, la maturation des cellules dendritiques induite par LPC.
Les capacités fonctionnelles de ces cellules (« contrôle », « Ciglitazone » et « LPC + Ciglitazone » à stimuler des lymphocytes T (LT) ont ensuite été analysées. Ces cellules ont été cultivées comme décrit dans l'exemple 4, en présence de lymphocytes T allogéniques purifiés. La sécrétion d'IFNy a été mesurée comme dans l'exemple 4.

**Tableau 8 - Réaction leucocytaire mixte : mesure de la sécrétion d'IFNγ par les lymphocytes T induite par les cellules dendritiques.**

| **Ratio DC/LT** | **0** | **0,05** | **0,1** | **0,2** |
|---|---|---|---|---|
| **Contrôle** | 0 | 54 ± 82 | 69 ± 4 | 364 ± 27 |
| **LPC** | 0 | 750 ± 184 | 899 ± 124 | 1326 ± 248 |
| **LPC + Ciglitizone** | 0 | 93 ± 7 | 169 ± 105 | 484 ± 229 |

*L'ensemble de ces résultats soulignent un rôle important des PPAR dans la maturation des cellules dendritiques induite par LPC ainsi que l'importance du rapport PPARγ*/ *PPAR δ dans la genèse de cellules dendritiques fonctionnellement matures. Ce rapport est modulé par le LPC et la ciglitizone.*

### Exemple 7 - Différenciation des monocytes en cellules dendritiques matures en présence de composés équivalents au LPC

Les inventeurs ont déterminé si des composés équivalents au LPC, c'est à dire impliquant les mêmes mécanismes d'action cellulaires via les mêmes récepteurs membranaires ou intracellulaire, pouvait également induire la différenciation des monocytes en cellules dendritiques matures.
Pour cela, les inventeurs ont recherché si l'ajout de PAF dans le milieu de culture pouvait augmenter l'action du LPC sur la différenciation des monocytes en cellules dendritiques matures.
Ainsi, une solution de PAF (Sigma, 5µM) a été ajoutée dans le milieu de culture au 5^{ème} jour de différenciation des monocytes. L'expression de CD86 des cellules " contrôle ", et des cellules " LPC ", obtenues en présence ou en absence de PAF, est analysée selon le protocole décrit dans l'exemple 2, et les résultats sont présentés dans le tableau 9.

**TABLEAU 9 - Expression du marqueur CD86 (intensité moyenne de fluorescence) des cellules " contrôles " et " LPC " en présence ou en absence de PAF**

| | **CD86** |
|---|---|
| **Contrôle** | 49,02 |
| **PAF** | 102,88 |
| **LPC** | 287,11 |
| **LPC + PAF** | 676,88 |

*Ces résultats suggèrent que le PAF seul induit une légère surexpression de CD86 contrairement au LPC qui induit une augmentation de l'expression de CD 86 de 470%. Par contre, il est important de noter que le PAF agit en synergie avec le LPC, puisque l'action des deux composés induit une augmentation de l'expression de CD86 de plus de 1200% .*

Afin de renforcer l'implication du récepteur au PAF, les inventeurs ont étudié l'action d'un antagoniste de ce récepteur sur la différenciation des monocytes en cellules dendritiques matures par le LPC.
Ainsi, une solution de l'antagoniste BN52021 du PAF (Biomol, Plymouth Meeting, USA, 100µM) a été ajoutée dans le milieu de culture au 5^{ème} jour de différenciation des monocytes. L'expression de CD86 des cellules " contrôles " et " LPC ", obtenues en présence ou en absence de BN52021, est analysée selon le protocole décrit dans l'exemple 2, et les résultats sont présentés dans le tableau 10.

**TABLEAU 10 - Expression de CD86 des cellules " LPC " en présence de l'antagoniste BN52021**

| | **CD86** |
|---|---|
| **LPC** | 100% |
| **LPC + BN52021** | 54% |

*Ces résultats suggèrent que l'action du LPC implique bien le récepteur du PAF mais suggèrent également que l'action du LPCpourrait impliquer d'autres récepteurs.*
A titre indicatif, les inventeurs ont également démontré que l'antagoniste BN52021 ajouté dans le milieu de culture au 5^{ème} jour de différenciation des monocytes en cellules dendritiques matures induites par des LDL oxydés induisait une inhibition de 100 % de l'action des LDL oxydés, suggérant la encore, que les mécanismes d'action du LPC et des LDL oxydés seraient différents.

### Exemple 8 - Stimulation in vivo de la réponse immune contre un antigène par le LPC

L'objectif de cet exemple est de montrer que le LPC est une molécule adjuvante du système immunitaire, pouvant être utilisée dans le cadre de l'immunisation pour augmenter la réponse T spécifique contre un antigène.

*Le LPC est un produit inflammatoire -* Dans cet exemple, une solution de LPC de 100 à 500 nmol dissout dans 50 µl de PBS sont injectés dans le coussinet plantaire de souris BALB/c (Charles River Laboratories) au jour 0. Les coussinet plantaires sont mesurés à l'aide d'un micromètre avant et après injection, jusqu'au 10^{ème} jour, et comparée aux coussinets plantaires de souris « contrôles », obtenus par injection de PBS (50 µl). L'intensité de l'inflammation est traduite par l'épaississement de la patte. La maximum d'épaississement est observé au 1^{er} jour, 24 h après l'injection.

**Tableau 11 - Inflammation du coussinet plantaire induite par le LPC épaississement de la patte après 24 h (en mm)**

| | **Epaississement de la patte (mm)** |
|---|---|
| **PBS** | 0,025 |
| **LPC 100 nmol** | 0,35 |
| **LPC 200 nmol** | 0,55 |
| **LPC 300 nmol** | 0,725 |
| **LPC 400 nmol** | 1,125 |
| **LPC 500 nmol** | 1,075 |

*Ces résultats montrent que la LPC induit une inflammation dépendante de la dose injectée.*

Afin de montrer que le LPC induit in vivo la maturation et donc la migration des cellules dendritiques vers les ganglions drainants, une solution de LPC (0,1 M dans le dibutyl-phtalate) a été appliquée sur la peau des oreilles de souris BALB/c (Charles River Laboratories), 10 minutes avant application d'une solution de FITC (Sigma) à 1,5 % en dibutyl-phtalate / acétone 1:1. Ce marqueur fluorescent est capturé par les cellules dendritiques de la peau. Après 24 h, les ganglions auriculaires et maxillaires sont prélevés et les cellules sont mises en suspension. La suspension cellulaire est enrichie en cellules dendritiques par centrifugation sur gradient de métrizamide (Sigma). Les cellules sont marquées par un anticorps (Pharmingen) reconnaissant les molécules de classe II du Complexe Majeur d'Histocompatibilité (CMH) et l'analyse en cytométrie de flux permet de quantifier le pourcentage de cellules de grande taille (cellules dendritiques), exprimant les molécules de classe II du CMH et contenant du FITC. Ces cellules sont des cellules présentatrices qui ont capturé le FITC en périphérie et ont migré vers les ganglions.

**Tableau 12- Stimulation de la migration des cellules dendritiques par le LPC**

| | **Cellules dendritiques FITC**^{**+**} |
|---|---|
| **FITC** | 7,1 ± 1,7 % |
| **LPC + FITC** | 19,3 ± 3,3 % |

*Ces résultats montrent que l'application de LPC stimule la migration des cellules dendritiques de la peau vers les ganglions drainants.*

*Le LPC stimule la réponse T spécifique d'un antigène soluble co-injecté.* Le LPC dissout en PBS (250 ou 500 nmol dans 50 µl) est mélangé au lysozyme d'oeuf de poule (HEL, 50 µg) et ce mélange est injecté dans le coussinet plantaire de souris BALB/c. Après 7 jours, les ganglions poplités sont prélevés, et leurs cellules sont restimulées en triplicat in vitro, dans un milieu de culture contenant 30 µM d'antigène HEL ou en l'absence d'HEL. Après 3 jours, la prolifération des lymphocytes T est mesurée par incorporation de thymidine tritiée pendant 16 h.

**Tableau 13 - Prolifération des cellules T spécifiques de HEL (incorporation de thymidine tritiée en CPM : moyenne des triplicats)**

| **Conditions d'immunisation** | **Prolifération en absence d'HEL** | **Restimulation par HEL (30 µM)** |
|---|---|---|
| **HEL** | 1315 ±275 | 5370 ± 1983 |
| **HEL + LPC 250 nmol** | 1608 ± 7 | 10885 ± 2861 |
| **HEL + LPC 500 nmol** | 2101 ± 87 | 23739 ± 4265 |

*Ces résultats montrent que le LPC co-injecté avec l'antigène favorise l'activation in vivo des lymphocytes T spécifiques de cet antigène.*

*Le LPC stimule la production d'anticorps spécifiques contre un antigène soluble co injecté.* Le LPC dissout en PBS (350 nmol dans 50 µl) est mélangé au lysozyme d'oeuf de poule (HEL, 1 mg/ml) et ce mélange est injecté dans le coussinet plantaire de souris BALB/c. Le lot de souris dite « HEL + LPC » reçoit 50µl de ce mélange dans les coussinets des deux pattes arrières. Le lot de souris dit « HEL » reçoit 50 µl de solution HEL (1 mg/ml en PBS) dans les deux pattes. Après 15 jours, une injection de rappel est réalisée en sous cutanée sur les 2 flancs. Le lot « HEL + LPC" reçoit sur chaque flanc 100 µl d'une solution de LPC (5 mM) et de HEL (1 mg/ml) en PBS. Le lot « HEL » reçoit sur chaque flanc 100 µl d'une solution de HEL (1mg/ml) en PBS. Deux semaines plus tard, le sang des souris est prelevé et les IgG spécifiques de l'antigène HEL sont dosées par ELISA par rapport à une gamme de solution standard d'IgG. Les courbes de titration donnant l'absorbance (Abs) en fonction du logarithme de la dilution du sérum sont représentées dans la figure 1. *Ces résultats suggèrent que le LPC co-injectée avec l'antigène favorise l'activation du système immunitaire et induit la synthèse d'anticorps spécifiques de l'antigène alors que l'injection de l'antigène seul n'induit pas de réponse. Le LPC induit une réponse humorale contre l'antigène, démontrant sa capacité d'adjuvant.*

*Le LPC peut induire une réponse des lymphocytes T CD8+ in vivo.* Dans cet exemple, un test d'hypersensibilité retardée de contact aux haptènes a été utilisé. Dans ce modèle, des souris BALB/c sont sensibilisées par application d'un haptène, le DNFB (1-fluoro-2,4-dinitrobenzene, solution à 0,5% en huile d'olive/acétone 1 :1), sur le dos. Cinq jours plus tard, une dose non irritante de DNFB (solution à 0,2% en huile d'olive/acétone 1 :1) est appliqué sur l'oreille gauche alors que l'oreille droite reçoit le solvant. Dans cette phase de révélation les lymphocytes T CD8⁺ spécifiques des protéines hapténisées sont recrutés et infiltrent l'oreille, sécrétant de l'IFNγ et produisant un oedème.

Un lot de souris dit " contrôle " a été sensibilisé par le DNFB seul et un autre lot (souris « LPC ») a reçu 500 nmol de LPC par application sur la peau du dos de 20 µl d'une solution éthanolique de LPC, 10 minutes avant l'application de DNFB. Cinq jours plus tard, les deux lots ont été traités de la même façon pour la phase de révélation et l'épaississement des oreilles a été mesuré à l'aide d'un micromètre 48 h après cette application.

**Tableau 14 - Mesure de l'oedème de l'oreille**

| | **Epaississement de l'oreille droite (µm)** | **Epaississement de l'oreille gauche (µm)** |
|---|---|---|
| **Contrôle** | 0 ± 4 | 67 ± 32 |
| **LPC** | 0±3 | 149±31 |

*Ces résultats montrent que l'application de LPC 10 minutes avant l'haptène lors de la phase de sensibilisation augmente l'oedème induit lors de la 2*^{*éme*} *application de l'haptène (révélation). Cela signifie que le LPC stimule la réponse relayée par les lymphocytes T CD8+.*

### Exemple 9 - Action d'une émulsion lipidique telle que l'Intralipid® sur la différenciation des monocytes en cellules dendritiques matures

Puisqu'il a été montré dans l'exemple 5 que une émulsion lipidique telle que l'Intralipid® bloque la genèse de cellules dendritiques matures induite par LPC, les inventeurs ont également recherché si ce blocage provenait d'une action indirecte de cette émulsion lipidique via une inhibition de l'action du LPC et/ou d'une action directe de cette émulsion lipidique.

*L'Intralipid® régule in vitro les PPAR.* Dans un premier temps, une solution d'Intralipid® (50 µg/ml de phospholipides) a été ajoutée dans le milieu de culture au 5^{ème} jour de différenciation des monocytes, selon un protocole comparable à celui décrit dans l'exemple 6. Les cellules ont été récoltées après 1 h, 2h ou 8h d'incubation avec l'Intralipid®, les protéines nucléaires ont été extraites et l'activité des PPARγ et PPARδ a été déterminée comme décrit dans l'exemple 6.

**Tableau 15 - Modulation de l'activité des PPARγ et PPAPδ des monocytes en cours de différenciation par l'Intralipid®**

| **Temps d'incubation avec l'Intralipid (h)** | PPARγ | PPARδ |
|---|---|---|
| **0** | 100 % | 100 % |
| **1** | 130% | 94% |
| **2** | 160 % | 94% |
| **8** | 170 % | 77% |

*Ces résultats montrent que l'Intralipid® seule, active PPARγ et inhibe l'activité de PPARδ. Cette action de l'Intralipid® est opposée à celle du LPC.*
Dans un deuxième temps, une solution d'Intralipid® (50 µg/ml de phospholipides) a été ajoutée dans le milieu de culture au 5^{ème} jour de différenciation des monocytes, 15 min avant l'ajout d'une solution de LPC (40 µM) (cellules dites « LPC + Intralipid »). Des cellules dites « Intralipid » ont également été obtenues en l'absence de LPC. Des cellules dites « LPC » ont été obtenues en l'absence d'Intralipid®. Les cellules ont été récoltées après 2h d'incubation, les protéines nucléaires ont été extraites et l'activité des PPARγ et PPARδ a été déterminée comme décrit dans l'exemple 6.

**Tableau 16 - L'Intralipid® bloque la génération de cellules dendritiques matures induite par LPC en modulant l'activité des PPARγ et PPARδ**

| | PPARγ | PPARδ |
|---|---|---|
| **Contrôle** | 100 % | 100 % |
| **Intralipid** | 156 % | 99 % |
| **LPC** | 1% | 160 % |
| **LPC + Intralipid** | 40 % | 80 % |

L'Intralipid® bloque également l'action du LPC en modulant l'activité des PPARγ et PPARδ.

*L'Intralipid® bloque in vivo l'inflammation et la réponse immune induite par LPC* Dans cet exemple, 3 lots de souris BALB/c sont immunisées contre l'antigène de lysozyme d'oeuf de poule (HEL) par injection de 50 µl de solution dans le coussinet plantaire. Le lot contrôle « HEL » reçoit 50 µg de HEL, le lot " HEL + Intralipid " reçoit un mélange d'Intralipid® (45 µl) et de 50 µg d'HEL (5 µl d'une solution à 10 mg/ml). Le lot " HEL + LPC " reçoit 50 µg d'HEL avec 350 nmol de LPC dissout en PBS. Le lot " HEL + LPC + Intralipid " reçoit 50 µg d'HEL mélangé à 350 nmol de LPC dissout dans 45 µl d'Intralipid®. Après 24 heures, l'épaisseur des coussinets est mesurée à l'aide d'un micromètre et comparée à la mesure faite avant injection. La différence d'épaisseur est proportionnelle à l'intensité de l'inflammation.

**Tableau 17 - L'Intralipid réduit l'inflammation induite in vivo par LPC**

| | **Epaississement moyen de la patte à 24 h (mm)** |
|---|---|
| **HEL + Intralipid** | 0,19 ± 0,04 |
| **HEL + LPC** | 1,07 ± 0,19 |
| **HEL + LPC + Intralipid** | 0,45 ± 0,04 |

*Ces résultats montrent que l'injection d'Intralipid® en même temps que le LPC réduit de 70 % l'inflammation du coussinet plantaire induite par LPC.*

Sept jours après l'injection, les ganglions poplités sont prélevés et leurs cellules sont restimulées in vitro en triplicat, dans un milieu de culture contenant 10 µM de HEL ou en l'absence d'HEL. Après 3 jours, la prolifération des lymphocytes T spécifiques de HEL est mesurée par incorporation de thymidine tritiée pendant 16 h.

**Tableau 18 - Prolifération des cellules T spécifiques de HEL (moyenne des souris)**

| **Conditions d'immunisation** | **Prolifération en absence d'HEL (cpm)** | **Restimulation par HEL (10 µM) (cpm)** |
|---|---|---|
| **HEL** | 370 ± 212 | 738 ± 408 |
| **HEL + LPC** | 2568 ± 1570 | 11340 ± 4426 |
| **HEL + LPC + Intralipid** | 1124 ± 541 | 3959 ± 2103 |

*Ces résultats montrent que l'Intralipid® injecté en même temps que le LPC bloque la stimulation de la réponse T induite par LPC.*
*L'ensemble de ces résultats montrent que l'Intralipid® bloque la genèse de cellules dendritiques matures induite par LPC, par une action indirecte de l'Intralipid® via une inhibition de l'action du LPC mais aussi par une action directe de l'Intralipid*® *sur les PPAR, antagonsite du LPC. Ces résultats montrent que l'Intralipid® seul a un rôle anti-inflammatoire.*
L'ensemble de ces résultats montre l'importance du rapport PPARγ / PPARδ dans la genèse de cellules dendritiques fonctionnellement matures. Ce rapport est modulé par le LPC, mais également, d'une façon inverse, par une émulsion lipidique telle que l'Intralipid® et la ciglitizone.

## Revendications

1. Utilisation d'au moins un inhibiteur de L-α-lysophosphatidylcholine pour la fabrication d'un médicament pour la prévention d'une inflammation et/ou pour lutter contre une maladie inflammatoire et/ou une maladie auto-immune dans laquelle l'inhibiteur est une émulsion lipidique comprenant des triglycérides, des phospholipides et du glycérol.

2. Utilisation selon la revendication 1 **caractérisé en ce que** l'émulsion lipidique comprend de 15 à 25% de triglycérides, de 0,5 à 1,5 % de phospholipides et de 1,8 à 2,6% de glycérol.

3. Utilisation d'au moins un inhibiteur de L-α-lysophosphatidylcholine pour la fabrication d'un médicament pour la prévention d'une inflammation et/ou pour lutter contre une maladie inflammatoire et/ou une maladie auto-immune dans laquelle l'inhibiteur est un agoniste des PPAR gamma
